(19) Europäisches Patentamt
European Patent Office
Office européen des brevets

(11) **EP 3 367 097 A1**

(12) **EUROPEAN PATENT APPLICATION**
published in accordance with Art. 153(4) EPC

(43) Date of publication:
**29.08.2018 Bulletin 2018/35**

(21) Application number: **16857452.3**

(22) Date of filing: **19.10.2016**

(51) Int Cl.:
**G01N 33/543** *(2006.01)*    **G01N 37/00** *(2006.01)*

(86) International application number:
**PCT/JP2016/080917**

(87) International publication number:
**WO 2017/069139 (27.04.2017 Gazette 2017/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.10.2015 JP 2015207343**

(71) Applicant: **Toyo Seikan Group Holdings, Ltd.**
**Shinagawa-ku**
**Tokyo 141-8627 (JP)**

(72) Inventors:
• **IWASAKI, Tsutomu**
  **Yokohama-shi**
  **Kanagawa 240-0062 (JP)**
• **OONUKI, Ryuuji**
  **Yokohama-shi**
  **Kanagawa 240-0062 (JP)**
• **KUNINORI, Masahiro**
  **Yokohama-shi**
  **Kanagawa 240-0062 (JP)**

(74) Representative: **Pestalozzi, Deborah**
**Isler & Pedrazzini AG**
**Giesshübelstrasse 45**
**Postfach 1772**
**8027 Zürich (CH)**

(54) **IMMUNOLOGICAL MEASUREMENT DEVICE**

(57) In the present invention, a strong capillary action is applied to a flowpath itself so as to reliably move and mix a specimen and a labeling substance, without constituting, by use of a porous material, the flowpath to move the specimen and without excessively prolonging nor complicating the flowpath. Furthermore, it is possible to reliably and effectively improve visibility and signal strength of the labeling substance without risk of clogging the flowpath even if a particle diameter of the labeling substance is increased. The present invention comprises a micro fluid device 1 comprising a flowpath which moves a specimen subjected to immunological measurement by an antigen-antibody reaction, a labeling reagent supply unit 4 that labels the specimen and is arranged in an upstream section of the flowpath, and a detection flowpath 8 arranged downstream than the labeling reagent supply unit 4 to measure the antigen-antibody reaction of the specimen, wherein the labeling reagent supply unit 4 comprises a labeling reagent having the labeling substance in which an antigen or an antibody is solidified, to label the specimen by the antigen-antibody reaction.

**EP 3 367 097 A1**

## EP 3 367 097 A1

**Description**

TECHNICAL FIELD

The present invention relates to an immunological measurement device which is used in immunological measurement by an antigen-antibody reaction, and particularly, to an immunological measurement device comprising a micro fluid device including a flowpath which moves a specimen subjected to an immunological measurement.

BACKGROUND ART

In general, there are known immunological measurement devices each of which is used in immunological measurement by an antigen-antibody reaction. As this type of immunological measurement device, for example, a device is known which is called an immunochromatographic device.

The immunochromatographic device enables immunological measurement utilizing an antigen-antibody reaction, and is principally used for detection of pathogens such as bacteria or viruses.

Specifically, in the immunochromatographic device, a specimen containing an analyte such as a blood or a humor is dropped onto a conjugate pad made of a nonwoven cloth or a fiber such as a glass fiber impregnated with a solidified labeling reagent containing an antibody or an antigen which reacts with the analyte (the antigen or the antibody) previously contained in the specimen. In consequence, if the antigen or the antibody is contained in the specimen, the antigen brings about an antigen-antibody reaction with the labeling reagent previously impregnated in the conjugate pad to form a complex, followed by labeling. Then, this complex moves in a membrane comprising a nitrocellulose foam which abuts on the conjugate pad, and is then captured by a capture reagent comprising a capture antibody arranged in a linear state in an upstream specimen capture section, so that a labeling substance visibly develops a color in a linear state.

Thus, presence/absence, concentration, kind and others of the specimen can be detected and measured on the basis of concentrations and positions (kinds) of lines of the labeling substance. Such types of devices are widely used as diagnosis kits for infection disease such as influenza and for allergy, commercially available pregnancy test kits and others.

As to the immunochromatographic devices used in such influenza test kits and the like, the devices having constitutions which enable easy handling and disposability are preferable. As a medium, i.e., a flowpath which moves the specimen and labeling reagent while mixing them to bring about the antigen-antibody reaction, for example, a membrane made of a porous material such as a nitrocellulose foam is used, and a capillary action by capillary phenomenon of the porous material is utilized, so that the specimen and the labeling reagent can be moved and progressed without requiring any pump power from the outside.

Furthermore, in the immunochromatographic device where a foam such as the nitrocellulose foam is used as the flowpath of the specimen, to avoid possibility that the labeling substance clogs porous areas of the foam which is the flowpath of the specimen, it has been necessary that a particle diameter of the labeling substance is sufficiently smaller than pores (spaces) of the porous material. For example, an average pore diameter of the nitrocellulose foam is about 10 $\mu$m, and in consideration of properties of the foam having various pore diameters, the size of the labeling substance is required to be 400 nm or less at the maximum, preferably around 40 nm. Therefore, in the immunochromatographic device, gold colloid having a particle diameter of 40 nm or less is used as the labeling substance.

However, in a case where the gold colloid having a particle diameter of 40 nm or less is used as the labeling substance, there is a problem that a specimen capture section of the device does not sufficiently develop a color because the particle diameter of the labeling substance is small and visibility (signal strength) deteriorates (worsens), even if the labeling reagent is captured by a capture reagent comprising the antigen or the antibody as a result of the antigen-antibody reaction. Particularly, when a concentration of an analyte such as viruses is low, the specimen capture section does not develop a color to a visible extent. Hence, even if the antigen is contained in the specimen, the result might be judged to be negative owing to the poor color development, and for example, an early infection of influenza might be overlooked. Therefore, when the immunochromatographic device is used, it is necessary to prepare the specimen containing the analyte at a high concentration, and such requirement might impinge invasiveness and a strong pain on a person to be tested.

To solve such a problem in the immunochromatographic device, for example, suggestions disclosed in Patent Literature 1 and Patent Literature 2 have been made.

Patent Literature 1 has suggested that as the labeling substance of the immunochromatographic device, minute gold colloid particles having an average particle diameter of 50 to 150 nm are used, and platinum is carried on the surfaces of the gold colloid particles. In this case, the gold colloid develops a red color and a platinum film having contrast of a black color is carried on the surfaces of the particles, and hence visibility/signal strength can be enhanced as compared with the usual gold colloid and the visibility of detection lines can be improved.

In addition, Patent Literature 2 has suggested that chambers (a sample injection chamber, a reaction chamber and a detection chamber) formed by photolithography are constituted as the flowpaths, without using a porous material such as a nitrocellulose foam as a flowpath to move a specimen in a test kit for use in a test of POC (point of care). This intends to avoid problems such as unevenness of pore diameters and difficulty of accurate manufacture which occur when the porous material is used.

RELATED ART DOCUMENTS

PATENT DOCUMENTS

[Patent Literature 1] Publication of Patent No. 3886000
[Patent Literature 2] PCT Application No. 2009-501908

SUMMARY OF THE INVENTION

However, according to the suggestion of Patent Literature 1, the gold colloid is coated with the platinum film which develops a black color to enhance the visibility/signal strength by a labeling substance in an immunochromatographic device, but a size (particle diameter) of the labeling substance scarcely changes as compared with the usual gold colloid. Therefore, the enhancement of the visibility/signal strength merely depends on a difference of the color development, and it is limited per se and any fundamental solution is not achieved.

That is to say, in the suggestion of Patent Literature 1, if it is intended to surely enhance the visibility/signal strength of the labeling substance by the immunochromatographic device, the particle diameter of the gold colloid which is the labeling substance has to be further increased as an only means. However, if the particle diameter of the gold colloid is increased, clogging occurs in the membrane made of the porous material, and the movement and development of the specimen stop, which problem is exactly the same as in conventional cases.

On the other hand, according to the suggestion of Patent Literature 2, as an immunological measurement device, an immunochromatographic device using a porous material such as a nitrocellulose foam is not employed as a medium which moves the specimen, and there is employed a micro fluid device where a chamber formed by photolithography on a substrate is used as a flowpath, which appears likely to avoid the clogging of the porous material in Patent Literature 1.

However, in the flowpath comprising the chamber disclosed in Patent Literature 2, a flowpath diameter of the chamber is required to be minute as much as possible to obtain a capillary phenomenon, and as a result, to enhance the visibility/signal strength, it is necessary to increase the particle diameter of the labeling substance as in Patent Literature 1. However, if the particle diameter of the labeling substance is increased, there is a problem that the clogging and development stop of the specimen in the minute chamber occur.

Furthermore, in the suggestion of Patent Literature 2, chambers (a sample injection chamber, a reaction chamber, and a detection chamber) constituting the flowpath are required to be very long to mix the specimen and the labeling substance, and the shape (pathway) of the flowpath is also required to be made into a complex shape such as a zigzag state or a comb tooth state. In consequence, there exists a problem that the flowpath is extremely long and complex.

The present invention has been suggested to solve the problems of conventional techniques as mentioned above, and an object of the present invention is to provide an immunological measurement device which can apply a strong capillary action and a mixing performance to a flowpath itself to surely move and mix a specimen and a labeling substance, without constituting, by use of a porous material, the flowpath to move the specimen and without prolonging nor complicating the flowpath, can avoid a risk of clogging in the flowpath even if a particle diameter of the labeling substance is increased, and can surely and effectively improve visibility and signal strength of the labeling substance.

SOLUTION TO PROBLEM

To achieve the above object, an immunological measurement device of the present invention comprises a micro fluid device including a flowpath to move a specimen which is subjected to an immunological measurement by an antigen-antibody reaction, and comprises a labeling reagent supply unit which is disposed in an upstream section of the flowpath and labels the specimen, and a detection flowpath which is disposed downstream than the labeling reagent supply unit and measures the antigen-antibody reaction of the specimen, wherein the labeling reagent supply unit comprises a labeling reagent which labels the specimen by the antigen-antibody reaction and contains the labeling substance having a solidified antigen or antibody.

ADVANTAGEOUS EFFECTS OF INVENTION

According to the immunological measurement device of the present invention, a strong capillary action and a mixing performance can be applied to a flowpath itself to surely move and mix a specimen and a labeling substance, without constituting, by use of a porous material, the flowpath to move the specimen and without prolonging nor complicating the flowpath.

Therefore, it is possible that a risk of clogging of the flowpath can be avoided even if the particle diameter of the labeling substance is increased, and visibility and signal strength of the labeling substance can surely and effectively be improved.

In consequence, there can be provided an immunological measurement device preferable for a particularly simple diagnosis kit for influenza.

BRIEF DESCRIPTION OF DRAWINGS

FIG. 1 is an appearance perspective view which shows a micro fluid device constituting an immunological measurement device concerned with one embodiment of the present invention;

FIG. 2 is an exploded perspective view showing the micro fluid device shown in FIG. 1;

FIG. 3 is a schematic plan view of a substrate for the micro fluid device shown in FIG. 1;

FIG. 4 is an explanatory view of a detection flowpath for the micro fluid device concerned with one embodiment of the present invention, (a) is a plan view of the detection flowpath, and (b) is a sectional view cut along the A-A line of the detection flowpath shown in (a);

FIG. 5 is an enlarged schematic plan view of a substantial part of one example of a capillary pump flowpath for the micro fluid device shown in FIG. 1;

FIG. 6 is an explanatory view explaining a pinning effect in the capillary pump flowpath shown in FIG. 5;

FIG. 7 is an explanatory view showing a behavior in which a liquid flows by a capillary action, in the capillary pump flowpath shown in Fig. 5;

FIG. 8 is an explanatory view showing an example in which a liquid sending direction is arbitrarily guided, in one example of the capillary pump flowpath concerned with the embodiment of the present invention;

FIG. 9 is an explanatory view showing a mixer flowpath in which a figure center of a flowpath section is continuously changed, in one example of the mixer flowpath of the micro fluid device shown in Fig. 1; (a) is an enlarged plan view of the mixer flowpath, (b) is an enlarged side view of a substrate section of the same mixer flowpath, and (c) is an appearance perspective view of the micro fluid device, and an area corresponding to the mixer flowpath shown in (a) and (b) is shown with a dotted line;

FIG. 10 is an enlarged plan view of a substantial part showing another example of the mixer flowpath of the micro fluid device shown in Fig. 1;

FIG. 11 is a sectional view cut along the B-B line of the mixer flowpath shown in Fig. 10;

FIG. 12 is a schematic perspective view of a downstream end of a groove disposed in the mixer flowpath shown in Fig. 10;

FIG. 13 is an enlarged plan view of a substantial part showing a modified example of the mixer flowpath shown in Fig. 10;

FIG. 14 is an explanatory view showing a state of the labeling substance which moves in a flowpath of the immunological measurement device concerned with one embodiment of the present invention; (a) is a plan view schematically showing the movement of a fluid in the flowpath, (b) and (c) are partially enlarged views of the flowpath shown in (a), (b) shows a case of the labeling substance concerned with the present invention and (c) shows a case of a conventional labeling substance;

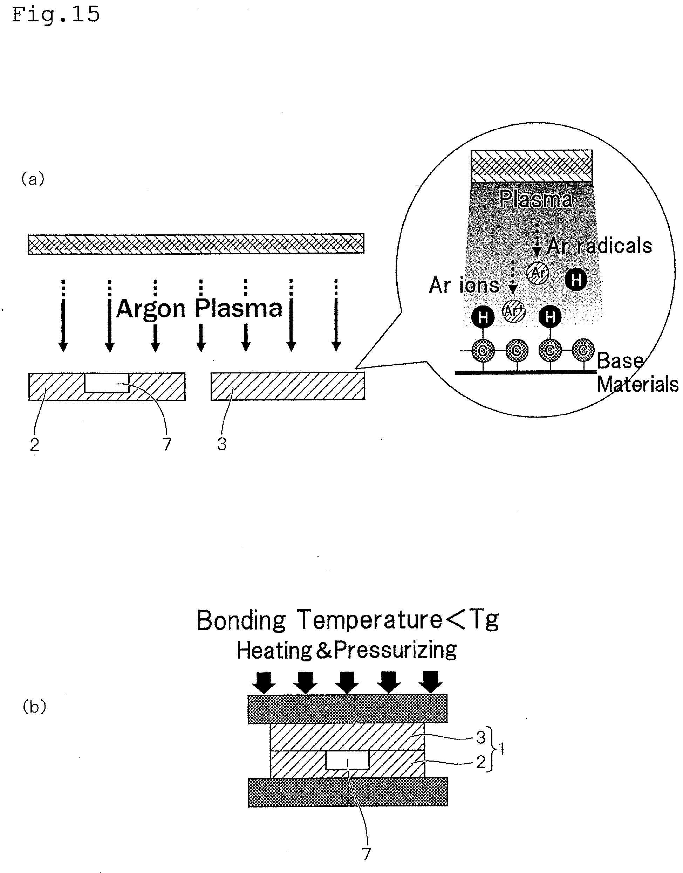

FIG 15 is an explanatory view schematically showing a manufacturing process of the micro fluid device which constitutes the immunological measurement device concerned with one embodiment of the present invention; (a) shows a step of applying an argon plasma to a bonded surface of two resin base materials constituting a substrate and a cover body of the micro fluid device to flatten and soften the bonded surface, and (b) shows a step of laminating the two resin base materials having the flattened and softened bonded surface, and then heating, pressurizing and bonding the two resin base materials.

DETAILED DESCRIPTION

Hereinafter, an embodiment of an immunological measurement device concerned with the present invention will be described with reference to the accompanying drawings.

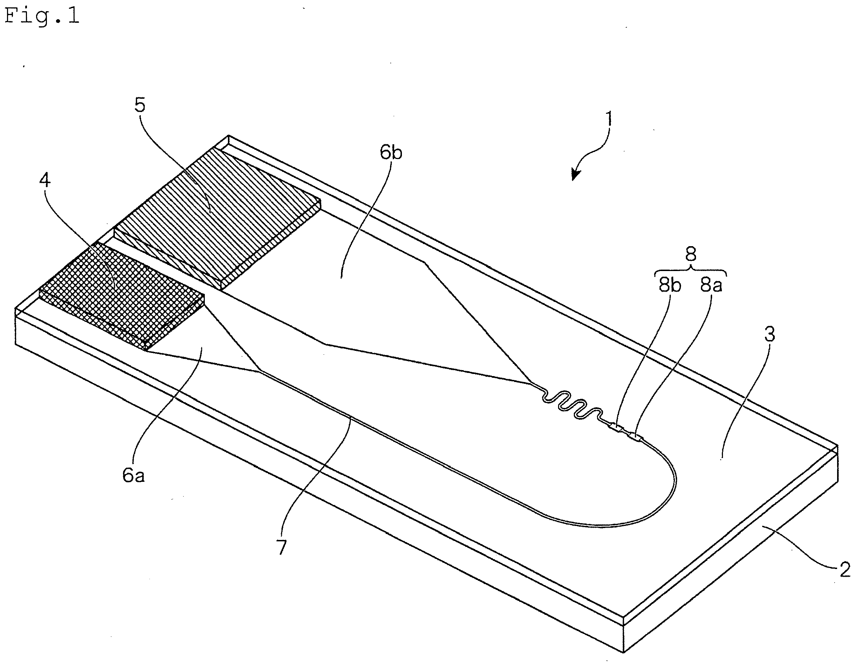

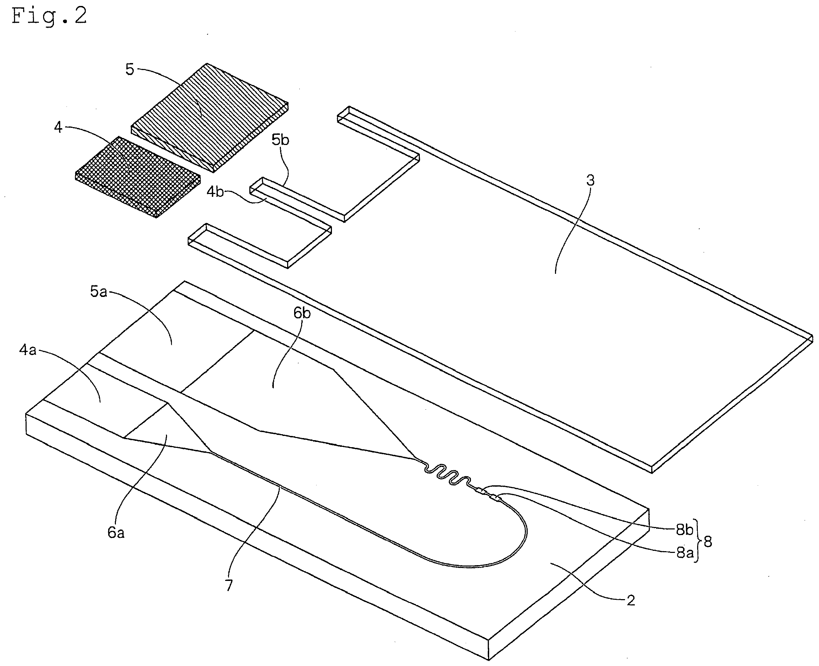

FIG. 1 is an appearance perspective view which shows a micro fluid device 1 constituting an immunological measurement device concerned with one embodiment of the present invention, and FIG. 2 is its exploded perspective view.

As shown in the drawings, an immunological measurement device concerned with the present embodiment comprises a micro fluid device 1.

[Micro Fluid Device]

As shown in FIGS. 1 and 2, a micro fluid device 1 concerned with the present embodiment comprises a capillary pump flowpath 6 to generate a capillary action, a mixer flowpath 7 connecting and communicating with the capillary pump flowpath to move a fluid while mixing, and a detection flowpath 8 connecting and communicating with the mixer flowpath 7 to measure an antigen-antibody reaction of a specimen. Therefore, the device 1 constitutes a passive type micro fluid device comprising a self liquid sending type flowpath which can move the fluid by the capillary action in the flowpath without requiring a pressure means to apply any pump power to the flowpath from the outside.

This passive type micro fluid device does not require a pressure means such as a pump, and hence the constitution of the device itself can be miniaturized and simplified. For example, the device can be a micro fluid device constituted as a simple and quick diagnosis kit for influenza.

Specifically, the micro fluid device 1 of the present embodiment is constituted as the diagnosis kit for influenza using an antigen-antibody reaction, and is constituted as the micro fluid device comprising a substrate 2 and a cover body (a lid member) 3 to cover the surface of the substrate 2.

In the present embodiment, on such a fluid device, there is formed a flowpath comprising the capillary pump flowpath 6, the mixer flowpath 7 and the detection flowpath 8, and in the most upstream section of the flowpath, there is disposed a conjugate pad 4 which becomes a labeling reagent supply unit onto which a specimen is dropped. Furthermore on the most downstream section of the flowpath, there is disposed an absorption pad 5 to absorb a residual fluid after analysis.

Furthermore, in the present embodiment, the conjugate pad 4 which is disposed in the upstream section of the flowpath and becomes the labeling reagent supply unit is impregnated with a labeling reagent including a labeling substance which labels an analyte contained in the dropped specimen by an antigen-antibody reaction and in which an antigen or an antibody is solidified, and the labeling substance is constituted so as to have a predetermined diameter or more (e.g., a diameter of 400 nm or more).

In the present embodiment, an influenza antigen is considered as the analyte to be contained in the specimen to be measured with the diagnosis kit for influenza, and the conjugate pad 4 is impregnated with the labeling reagent comprising the labeling substance in which a labeling antibody combining with the influenza antigen is solidified.

Additionally, the labeling substance contained in the labeling reagent comprises large-diameter beads having a predetermined diameter or more.

In the micro fluid device 1 of the present embodiment, as described later, the respective flowpaths 6, 7 and 8 which move and send the specimen and the labeling reagent are constituted to have a sufficient flowpath width and a sufficient flowpath depth so that clogging does not occur in the flowpaths even if a particle diameter of the labeling substance is increased, while maintaining respective flowpath functions. Consequently, the labeling substance having a large particle diameter can noticeably improve the visibility and signal strength of the specimen in an antigen-antibody reaction, and the antigen-antibody reaction of the specimen can easily and reliably be measured.

Hereinafter, description will be made as to constitutions of respective parts of the micro fluid device 1 of the present embodiment which are capable of moving and sending the labeling substance having a large diameter.

[Substrate and Cover Body]

As shown in FIG. 2, in the surface of the substrate 2, there are formed the respective parts of a conjugate pad abutting part 4a, a first capillary pump flowpath 6a, the mixer flowpath 7, the detection flowpath 8, a second capillary pump flowpath 6b and an absorption pad abutting part 5a.

Specifically, in the substrate 2 made of glass or plastic, for example, flowpaths (the capillary pump flowpath 6, the mixer flowpath 7 and the detection flowpath 8) which are micro flowpath spaces having a width of about 100 μm and a depth of about 50 μm are formed by transfer, engraving or the like, and the cover body 3 which is the lid member is bonded to the upper surfaces of the flowpaths, to form micro flowpaths which constitute a reaction field of the antigen-antibody reaction. It is to be noted that a flowpath size (a width and a depth), a flowpath length, a flowpath shape and others of the flowpaths 6, 7 and 8 formed in the substrate can arbitrarily be set in accordance with a use application of the micro fluid device 1, a kind of fluid, and the like.

Furthermore, in the present embodiment, to move and send the after-mentioned large-diameter labeling substance, each of the flowpaths 6, 7 and 8 is formed so that its size (the flowpath width and the flowpath depth) is larger than the diameter of the labeling substance.

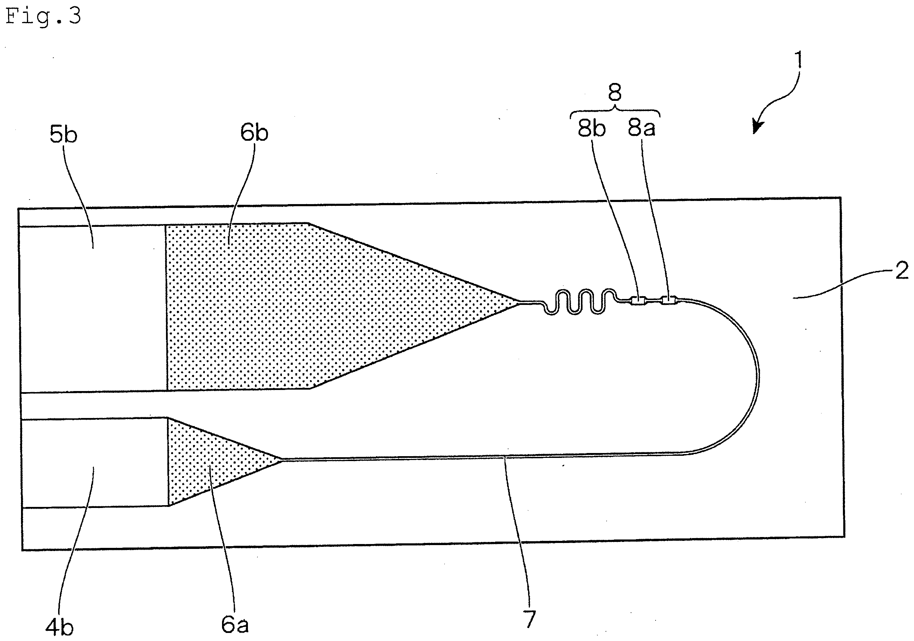

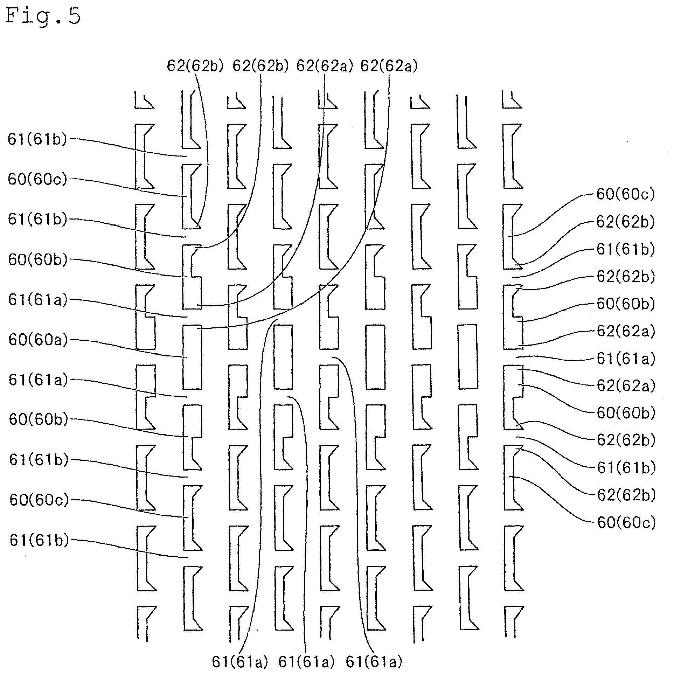

FIG. 3 is a schematic plan view of the substrate 2. It is to be noted that regions shown with dots in the drawing are micro liquid sending structures (see FIGS. 5 to 8) constituting the capillary pump flowpath 6 which will be described later.

In the substrate 2, the flowpaths can be formed in micro shapes by the transfer or the like. For example, the substrate can be formed by casting of an ultraviolet curable, thermosetting or two-liquid curable resin such as polydimethylsiloxane. Alternatively, the substrate may be formed by injection, nanoimprint or the like using a thermoplastic resin such as polymethyl methacrylate, polystyrene, polycarbonate, cycloolefin copolymer or cycloolefin polymer.

Alternatively, the substrate may be formed by etching, ultraprecision machining or the like using glass or silicon.

It is preferable that the cover body 3 can be made of a resin or glass and is transparent to such an extent that the detection flowpath 8 formed in the surface of the substrate 2 can be seen through the cover body.

As shown in FIG. 2, the cover body 3 is bonded to the substrate 2 to seal the first capillary pump flowpath 6a, the mixer flowpath 7, the detection flowpath 8 (a test flowpath 8a and a control flowpath 8b) and the second capillary pump flowpath 6b while holding the conjugate pad 4 and the absorption pad 5 in cutout parts 4b and 5b formed on one end side, respectively.

At this time, the conjugate pad abutting part 4a is formed to be as deep as or to be slightly deeper than the bottom surface of the first capillary pump flowpath 6a so that the first capillary pump flowpath 6a sealed with the cover body 3 has an opening on the side of the conjugate pad abutting part 4a. Consequently, the conjugate pad 4 is connected to the first capillary pump flowpath 6a via the opening.

Similarly, the absorption pad abutting part 5a is formed to be as deep as or to be slightly deeper than the bottom surface of the second capillary pump flowpath 6b so that the second capillary pump flowpath 6b sealed with the cover body 3 has an opening on the side of the absorption pad abutting part 5a. Consequently, the absorption pad 5 is connected to the second capillary pump flowpath 6b via the opening.

Additionally, description will be made later as to a bonding method of the substrate 2 and the cover body 3 which are made of the resin with reference to FIG 15.

The capillary pump flowpath 6 comprises two flowpaths, i.e., the first capillary pump flowpath 6a on an upstream side which is continuous with the conjugate pad 4, and the second capillary pump flowpath 6b on a downstream side which is continuous with the absorption pad 5. Each of the first capillary pump flowpath 6a and the second capillary pump flowpath 6b comprises the micro liquid sending structure utilizing a capillary phenomenon as a driving force to send a preparation containing the specimen which is an analysis target and the labeling reagent.

Consequently, the fluid containing the specimen and the labeling reagent is moved and sent in the flowpaths only by the driving force of the capillary pump flowpath 6 without requiring a pressure means such as an external pump, and hence the passive type micro fluid device preferable for the diagnosis kit for influenza can be constituted.

Furthermore, in the present embodiment, to move and send the after-mentioned large-diameter labeling substance while maintaining the function of the capillary pump, the capillary pump flowpath 6 is formed in a width and a depth of, for example, 30 $\mu$m or more so that the flowpath width and flowpath depth of the capillary pump flowpath are sufficiently larger than the diameter (e.g., 400 nm or more) of the labeling substance.

Description will be made later as to details of the capillary pump flowpath 6 concerned with the present embodiment with reference to FIGS. 5 to 8.

The mixer flowpath 7 connects and communicates between the first capillary pump flowpath 6a and the detection flowpath 8, and constitutes a flowpath space to efficiently mix the fluid containing the specimen and the labeling reagent and sent by the driving force of the capillary pump flowpath 6.

The fluid can efficiently, quickly and reliably be mixed while moving in the mixer flowpath 7, and the specimen and the labeling reagent can reliably be combined and reacted until reaching the detection flowpath 8.

Furthermore, in the mixer flowpath 7 concerned with the present embodiment, it is possible to move the fluid while suppressing an increase of a loss head or pressure loss in the flowpath down to the same amount as in a case where the mixer flowpath is not disposed but a straight flowpath is disposed, and the flowpath can pass and move the fluid while mixing the fluid, without requiring any pressure means such as the external pump other than the driving force by the capillary pump flowpath 6.

Consequently, the mixer flowpath can be utilized as the most suitable flowpath for the passive type micro fluid device constituting, for example, the diagnosis kit for influenza.

Additionally, to move and send the large-diameter labeling substance concerned with the present embodiment, the mixer flowpath 7 is also formed in a width and a depth of, for example, 30 $\mu$m or more so that the flowpath width and the flowpath depth of the mixer flowpath 7 are sufficiently larger than the diameter (e.g., 400 nm or more) of the labeling substance.

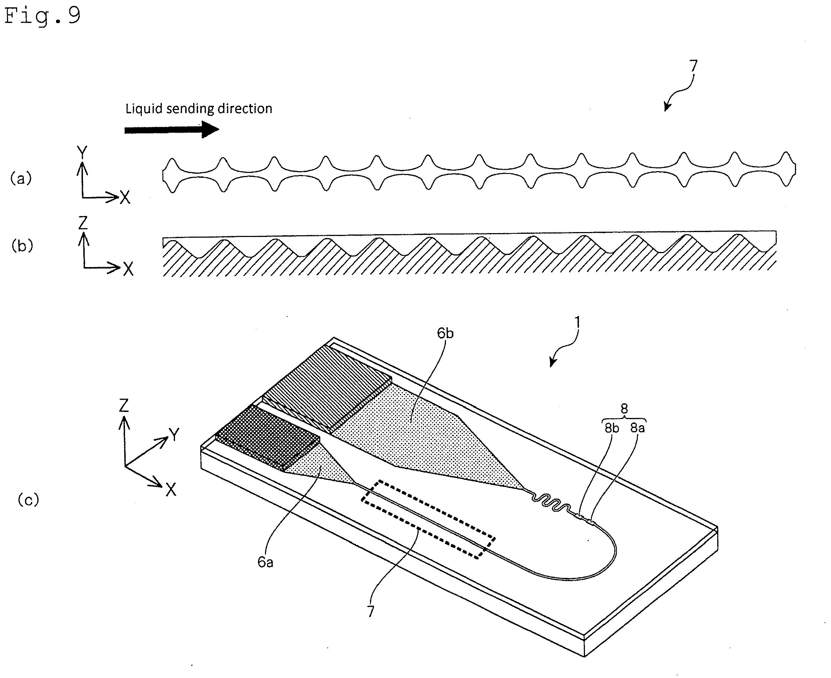

Description will be made later as to details of the mixer flowpath 7 concerned with the present embodiment with reference to FIGS. 9 to 13.

The detection flowpath 8 is a flowpath space formed on the flowpath on the downstream side of the mixer flowpath 7, and constitutes a detecting section with which a concentration of the analyte contained in the specimen by the antigen-antibody reaction is visually recognized and measured.

The detection flowpath 8 of the present embodiment comprises the test flowpath 8a coated with a capture antibody which captures the influenza antigen combined with the antibody of the labeling reagent by the antigen-antibody reaction, and the control flowpath 8b coated with a capture antibody to capture the labeling reagent which does not combine with the influenza antigen.

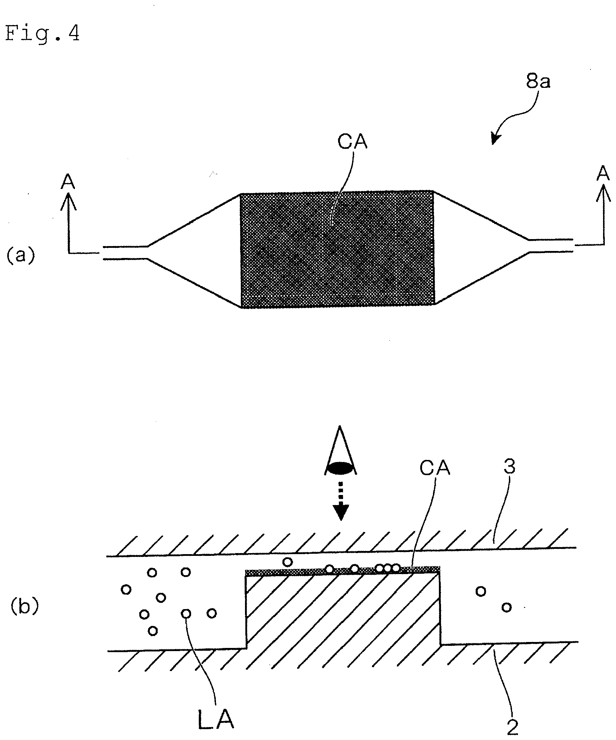

FIG 4 is an explanatory view of the test flowpath 8a (or the control flowpath 8b) of the detection flowpath 8 for the micro fluid device 1 concerned with the present embodiment, (a) is a plan view of the flowpath, and (b) is a sectional view cut along the A-A line shown in (a).

As shown in the drawings, the test flowpath 8a of the detection flowpath 8 is formed to have a large width and a small depth, and its bottom surface is coated with a capture reagent CA comprising the capture antibody, so that a complex (the influenza antigen combined with the labeling antibody of the labeling reagent) LA is captured by the antigen-antibody reaction. Although not particularly shown in the drawings, the control flowpath 8b also has a similar constitution.

Furthermore, as shown in FIG 4(b), the labeling substance of the complex LA captured in the detection flowpath 8 is visually recognized and confirmed, to perform judgment, detection or the like of its concentration.

Also as to the detection flowpath 8, similarly to the mixer flowpath 7 mentioned above, to move and send the large-diameter (e.g., a diameter of 400 nm or more) labeling substance concerned with the present embodiment, the detection flowpath is formed in a width and a depth of, for example, 10 $\mu$m or more so that the flowpath width and the flowpath depth are sufficiently larger than the diameter of the labeling substance.

When it is diagnosed whether or not a person to be tested is infected with influenza by use of the micro fluid device 1 mentioned above, a specimen preparation containing a specimen (an analysis target) such as a nasal swab sampled from the person to be tested is initially dropped onto the conjugate pad 4.

In this case, an unshown sample pad may be disposed to abut on an upper part of the conjugate pad 4, and the specimen preparation may be dropped onto the sample pad, to supply the specimen preparation to the conjugate pad 4 via the sample pad. The sample pad is, for example, filter paper made of a glass fiber, and is disposed for the purpose of filtering impurities contained in the specimen preparation, or for pH adjustment of the specimen preparation when the pad is impregnated with a buffer having a high buffering capacity.

The specimen preparation dropped onto the conjugate pad 4 exudes together with the labeling reagent impregnated in the conjugate pad 4 to enter the first capillary pump flowpath 6a. Then, the specimen preparation proceeds in the first capillary pump flowpath 6a and is sent to the mixer flowpath 7 by use of the capillary phenomenon as the driving force.

The specimen preparation and labeling reagent sent to the mixer flowpath 7 are mixed and kneaded in the mixer flowpath 7, a part of the mixture is reacted to form a complex, and by the driving force of the capillary pump flowpath 6, the mixture is moved and sent to the detection flowpath 8 formed on a flowpath on the downstream side of the capillary pump flowpath.

In the detection flowpath 8, the complex formed in the mixer flowpath 7 is captured, and the concentration of the analyte which appears in the labeling substance is visually recognized and measured.

On reaching the second capillary pump flowpath 6b, the residual fluid of the specimen preparation passed through the detection flowpath 8 utilizes the capillary phenomenon of the flowpath as the driving force to proceed in the second capillary pump flowpath 6b.

At this time, when a flow length of the specimen preparation increases, a flow resistance accordingly increases cumulatively to decrease a liquid sending speed of the specimen preparation. However, when the second capillary pump flowpath 6b is formed to widen toward its end as shown in FIGS. 1 to 3 and when the number of liquid sending passages 61 each of which is formed between microprojections 60 in the after-mentioned micro liquid sending structure is increased along a flow direction, it is possible to inhibit drop of the liquid sending speed due to the flow resistance.

In consequence, the specimen preparation can be sent at a constant flow rate without using the external pump or the like, which enables highly reproducible analysis (diagnosis).

The residual fluid of the specimen preparation proceeds in the second capillary pump flowpath 6b and is then absorbed by the absorption pad 5.

Thus, when a patient is infected with influenza, the specimen preparation contains the influenza antigen, and when the specimen preparation is dropped onto the conjugate pad 4, the labeling reagent impregnated in the conjugate pad 4 elutes into the specimen preparation. Afterward, a part of the specimen preparation combines with the influenza antigen by the antigen-antibody reaction, and is sent to the detection flowpath 8 formed on a downstream flowpath of the mixer flowpath 7.

As described above, the detection flowpath 8 comprises the test flowpath 8a coated with the capture reagent comprising the capture antibody, and the control flowpath 8b coated with the capture reagent comprising the capture antibody to capture the labeling reagent which does not combine with the influenza antigen. Therefore, when the color developed by the labeling substance is visually recognized only in the control flowpath 8b after the specimen preparation is passed through the detection flowpath 8, it can be diagnosed that the person to be tested is not infected with influenza, and when the color developed by the labeling substance is visually recognized also in the test flowpath 8a, it can be diagnosed that the person to be tested is infected with influenza.

Furthermore, in the present embodiment, the flowpath width and flowpath depth of each of the flowpaths 6, 7 and 8 to move and send the specimen and the labeling reagent are set to be sufficiently larger than the diameter of the labeling substance, while maintaining a performance of each flowpath. Additionally, as the labeling substance in which the labeling antibody combining with the influenza antigen contained in the dropped specimen is solidified, the labeling substance having a predetermined diameter or more is usable.

As a result, suitable visibility and signal strength are obtainable by the labeling substance having a particle diameter which is sufficiently larger than that of, for example, gold colloid used as a labeling substance in an immunochromatographic device, and a judgment accuracy of influenza infection can be enhanced.

Additionally, in the present embodiment, there has been described the example where the conjugate pad 4 is used as the labeling reagent supply unit, but the unit is not limited to this example. Alternatively, a wall surface of a flowpath disposed upstream than the detection flowpath 8, e.g., the mixer flowpath 7 or a flowpath disposed upstream than the mixer flowpath 7 may be coated with the labeling reagent, dried and immobilized, and an area corresponding to the wall surface may be used as the labeling reagent supply unit.

In this case, when the specimen preparation flowing inside from a specimen intake port disposed in an upstream section of the device passes through the area, the labeling reagent is dissolved and flows downstream together with the specimen preparation.

[Capillary Pump Flowpath]

Next, description will be made as to the micro liquid sending structure of the capillary pump flowpath 6 which utilizes the capillary phenomenon as the liquid sending driving force to send the preparation containing the analysis target in the micro fluid device 1 concerned with the present embodiment mentioned above.

FIG. 5 is an enlarged schematic plan view of a substantial part of one example of the micro liquid sending structure of the capillary pump flowpath 6 concerned with the present embodiment.

In the micro liquid sending structure constituting the capillary pump flowpath 6 shown in the drawing, a plurality of microprojections 60 are arranged in one row, and unit rows including the liquid sending passages 61 each of which is disposed in a space between adjacent microprojections 60 are periodically arranged so that the space between the microprojections 60 is a space to bring about the capillary phenomenon (e.g., the space between the adjacent microprojections 60 in one unit row is from 1 to 1000 $\mu$m and a space between adjacent unit rows is from 1 to 1000 $\mu$m).

It is to be noted that in the example shown in FIG. 5, for example, each of the microprojections 60 has a longitudinal size of 120 $\mu$m, a lateral size of 30 $\mu$m and a height of 30 m, the space between the adjacent microprojections 60 in the one unit row is set to 30 $\mu$m, and the space between the adjacent unit rows is set to 60 $\mu$m.

In the micro fluid device 1 mentioned above, the height of each of the microprojections 60 corresponds to a depth of the bottom surface of each of the first capillary pump flowpath 6a and the second capillary pump flowpath 6b each comprising the micro liquid sending structure concerned with the present embodiment, and tips of the microprojections 60 come in contact closely with the cover body 3.

Consequently, spaces of peripheries of the microprojections 60 are sealed with the cover body 3.

However, the present invention is not limited to the constitution where the microprojections 60 are closely in contact with the cover body 3. For example, a space may be present between the tip of each of the microprojections 60 and the cover body 3 and a capillary action may further be generated in this space.

Furthermore, in the present embodiment, the microprojections 60 are uniformly arranged so that the liquid sending passage 61 formed in one unit row and the liquid sending passage 61 formed in a unit row adjacent to the above unit row are alternately positioned as shown in the drawing. When a large number of liquid sending passages 61 are thus arranged in the form of a parallel circuit, it is possible to generate the capillary action in proportion to the number of the liquid sending passages while inhibiting the increase of the flow resistance, and even when a size of each liquid sending passage is set to be large, it is possible to generate the capillary action required in moving and sending the specimen fluid and the labeling reagent.

Additionally, in the example shown in FIG. 5, in each of the microprojections 60 which are adjacent to each other via the liquid sending passage 61, an edge portion 62 which develops a pinning effect is formed on an outlet side of each liquid sending passage 61.

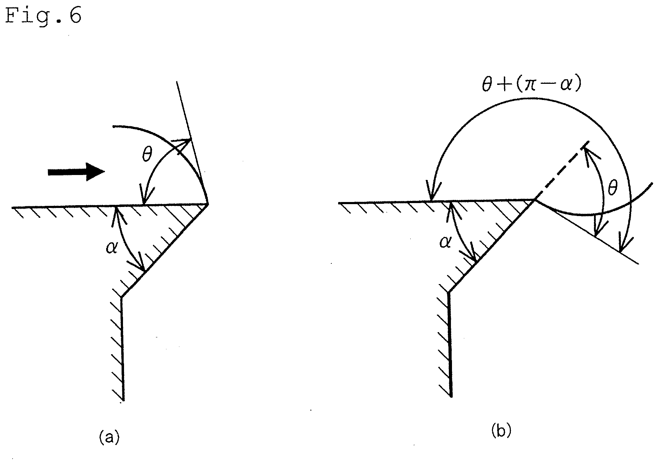

Here, as shown in FIG 6, the pinning effect indicates a phenomenon where on reaching the edge portion (see FIG. 6(a)), a liquid surface progressed at a contact angle $\theta$ along a plane cannot ride across the edge portion until the contact angle becomes $\theta + (\pi\text{-}\alpha)$, in which $\alpha$ is an angle formed by the plane and an outer surface of the edge portion (see FIG. 6(b)). In the present embodiment, the angle $\alpha$ formed by the plane and the outer surface of the edge portion at this time is defined as a pinning angle.

In the example shown in FIG. 5, the pinning angle of the edge portion 62 formed on the outlet side of the liquid sending passage 61 is suitably set. Consequently, at least one of the liquid sending passages 61 each of which is formed between the microprojections 60 in the one unit row is considered as a low flow resistance liquid sending passage 61a in which the flow resistance is relatively decreased to be lower than that of another liquid sending passage 61b.

That is to say, the pinning angle of an edge portion 62b formed on an outlet side of the liquid sending passage 61b between a microprojection 60b and a microprojection 60c adjacent to each other via the liquid sending passage 61 b other than the low flow resistance liquid sending passage 61a is set to be smaller than the pinning angle of an edge portion 62a formed on an outlet side of the low flow resistance liquid sending passage 61a between a microprojection 60a and the microprojection 60b adjacent to each other via the low flow resistance liquid sending passage 61a. Consequently, the flow resistance of the low flow resistance liquid sending passage 61a is relatively decreased to be lower than the flow resistance of the other liquid sending passage 61b.

It is to be noted that in the example shown in FIG 5, the microprojection 60a is formed in a rectangular shape, and the edge portions 62a having a pinning angle of 90° are formed at both ends of the microprojection, respectively. Furthermore, the edge portion 62a having the pinning angle of 90° is formed at one end of the microprojection 60b, and the edge portion 62b having a pinning angle of 45° is formed at the other end of the microprojection, whereas the edge portions 62b having the pinning angle of 45° are formed at both ends of the microprojection 60c, respectively. Tips of the edge portions 62a and 62b may be rounded to such an extent that the development of the pinning effect is not disturbed.

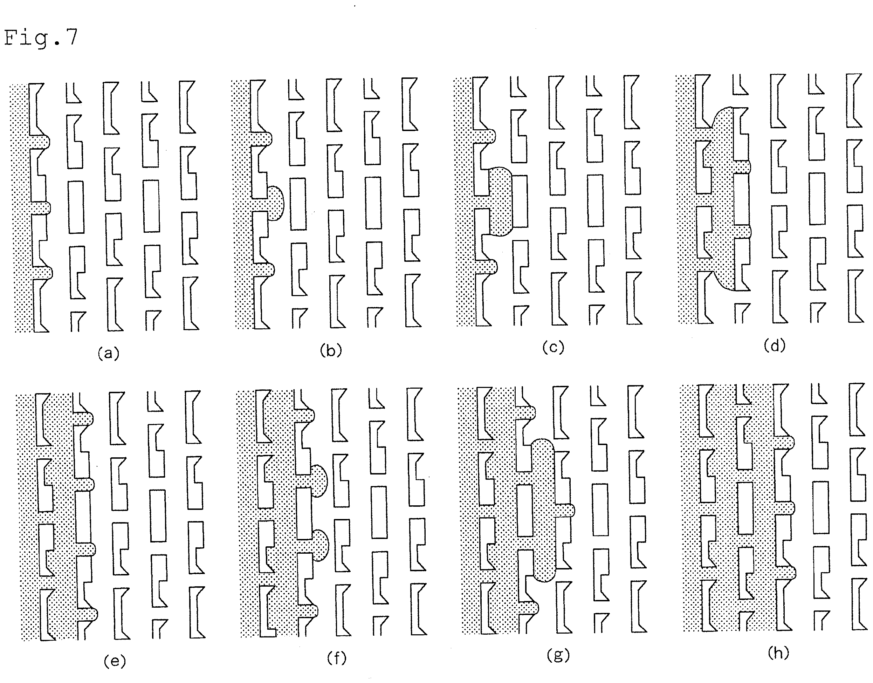

Furthermore, in the example shown in FIG. 5, the microprojections 60a, 60b and 60c are arranged in each unit row so that at least one of the liquid sending passages 61 is defined as the low flow resistance liquid sending passage 61a in which the flow resistance is relatively decreased to be lower than that of the other liquid sending passage 61 b, and so that the low flow resistance liquid sending passages 61a are arranged along a predetermined liquid sending direction. In this case, as shown in FIG. 7, a liquid preferentially passes through the low flow resistance liquid sending passage 61a in which the flow resistance is low (see FIGS. 7(a) to (c)), starts to spread from this passage to a space between the unit rows owing to the capillary phenomenon (see FIGS. 7(c) to (e)), and repeats flowing in this way (see FIGS. 7(e) to (h)). Consequently, flow properties in utilizing the capillary phenomenon to send the liquid can suitably be controlled in high reproducibility without causing any unevenness in the liquid sending direction.

Additionally, in the micro fluid device 1 mentioned above, each of the first capillary pump flowpath 6a and the second capillary pump flowpath 6b is formed in an isosceles triangular shape, and the low flow resistance liquid sending passages 61a are arranged along a perpendicular line drawn from a vertex of the shape down to a bottom side thereof. Consequently, the driving force to send the liquid utilizing the capillary phenomenon by the micro liquid sending structure is efficiently obtainable, but the arrangement of the low flow resistance liquid sending passages 61 a can suitably be set in accordance with a desirable liquid sending direction.

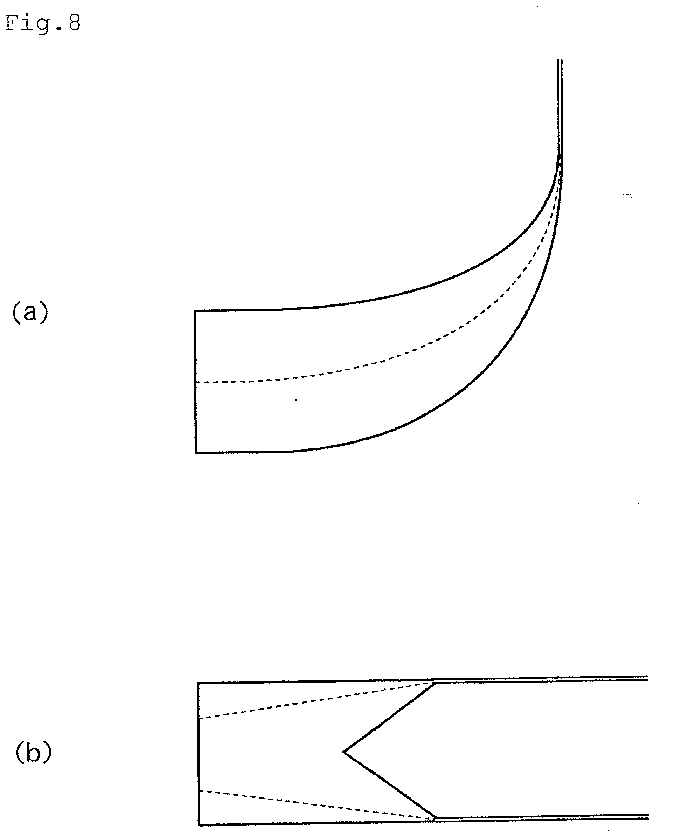

For example, in a case of curving the liquid sending direction as shown in FIG. 8(a) or in a case of branching the liquid sending direction into two directions or more as shown in FIG. 8(b), the low flow resistance liquid sending passages 61a are arranged along a direction shown with a dotted line in the drawing, and hence, the liquid sending direction can be guided to an arbitrary direction.

As described above, according to the capillary pump flowpath 6 concerned with the present embodiment, a strong driving force to send the liquid is obtainable by the micro liquid sending structure where the plurality of microprojections are arranged via the spaces to cause the capillary phenomenon. Furthermore, when the driving force is obtained, the liquid sending direction can arbitrarily be controlled in the high reproducibility without causing any unevenness in the liquid sending direction.

Furthermore, according to the capillary pump flowpath 6 comprising this constitution, the strong driving force is obtainable by the micro liquid sending structure, and hence minute chambers are not required to be formed along a complicated long pathway to obtain a capillary operation as disclosed in Patent Literature 2 mentioned above, so that a size (a width and a depth) of the flowpath can be set to be large.

Therefore, in the present embodiment, for example, each of the flowpath width and the flowpath depth is set to a length (a size) of 30 $\mu$m or more so that the flowpath width and flowpath depth of the capillary pump flowpath 6 are sufficiently larger than the diameter of the labeling substance. For example, even when the labeling substance having the large diameter of 400 nm or more is employed as the labeling substance, clogging with the labeling substance and slowdown, stop or the like of the liquid sending do not occur in the capillary pump flowpath 6.

Consequently, the labeling substance having the large diameter can enhance its visibility and signal strength in the antigen-antibody reaction, without risk of clogging the capillary pump flowpath 6 even if the particle diameter of the labeling substance is increased.

It is to be noted that the flowpath width and flowpath depth of the capillary pump flowpath 6 are not required to be 30 $\mu$m or more in all areas, as long as the flowpath width and the flowpath depth are larger than at least the diameter of the labeling substance. However, it is more preferable that the flowpath width and the flowpath depth are 30 $\mu$m or more in all the areas of the capillary pump flowpath 6.

[Mixer Flowpath]

Next, description will be made as to the mixer flowpath 7 to efficiently mix the fluid containing the specimen and the labeling reagent and sent by the driving force of the capillary pump flowpath 6 in the micro fluid device 1 of the present embodiment.

In an immunological measurement device such as the diagnosis kit for influenza, it is important to reliably mix, combine and react the influenza antigen contained in the specimen and the labeling reagent (the labeling substance having the solidified labeling antibody) in the mixer flowpath 7 toward the detection flowpath 8.

That is to say, as described above in the detection flowpath 8, the influenza antigen combined with the antibody solidified in the labeling substance is captured to visually recognize and judge presence/absence of influenza infection, and the influenza antigen which does not combine or react with the labeling reagent does not contribute to the diagnosis.

Therefore, in the mixer flowpath 7 toward the detection flowpath 8, it is necessary to reliably combine and react the influenza antigen and the labeling reagent.

Furthermore, from a viewpoint of decreasing the flow resistance to reliably perform self liquid sending, it is desirable to shorten the flowpath length of the mixer flowpath 7 as much as possible. For this purpose, it is important to reliably and efficiently mix and react two fluids (the influenza antigen (the specimen) and the antibody (the labeling reagent)) in the mixer flowpath 7.

Consequently, in the present embodiment, the micro fluid device 1 constituting the above-mentioned diagnosis kit for influenza comprises a flowpath having the following constitution as the mixer flowpath 7 to mix and combine target fluids (the specimen (the influenza antigen) and the labeling reagent).

Hereinafter, description will be made as to details of the mixer flowpath 7 concerned with the present embodiment with reference to FIGS. 9 to 13.

[Figure Center Change Mixer Flowpath]

The mixer flowpath 7 is a flowpath space of the micro fluid device 1 which moves the fluid while mixing the fluid. In an example shown in FIG. 9, for the purpose of reliably and efficiently mixing a plurality of fluids to be moved and sent, the mixer flowpath comprises a periodic repetition structure where a figure center of a flowpath section changes along a flowpath axial direction and fluctuation in loss head due to change of a sectional shape is not generated.

FIG. 9 is an explanatory view showing the mixer flowpath in which the figure center of the flowpath section is continuously changed, in one example of the mixer flowpath 7 of the micro fluid device 1 of the present embodiment; (a) is an enlarged plan view of the mixer flowpath, (b) is an enlarged side view of a substrate section of the same mixer flowpath, and (c) is an appearance perspective view of the micro fluid device, and an area corresponding to the mixer flowpath shown in (a) and (b) is shown with a dotted line.

The mixer flowpath 7 shown in FIG 9 has the sectional shape of the flowpath which is formed so that a position of the figure center of the flowpath section repeatedly changes periodically at regular intervals along the flowpath axial direction.

Here, "the figure center" is the center of a subject plane figure, i.e., a position of the center of gravity. For example, when the subject figure is rectangular, an intersection of diagonal lines corresponds to the figure center.

In the present embodiment, the plane figure constituting the flowpath section of the mixer flowpath 7 is considered as a subject, and the mixer flowpath is formed to displace the figure center along the axial direction of the flowpath.

Thus, the position of the figure center of the flowpath section is changed along the flowpath axial direction, and hence, a velocity of the fluid flowing in the flowpath is generated and changed in a vertical direction or a horizontal direction of the flowpath section in accordance with the change of the figure center of the flowpath section.

In consequence, the fluid in the flowpath is mixed and kneaded in accordance with a direction or size of a component of the velocity. When the figure center periodically changes repeatedly in a plurality of directions, the fluid is also repeatedly kneaded and stirred, and hence a plurality of fluids can efficiently and reliably be mixed even in a short flowpath.

However, when the figure center of the flowpath section is simply changed, the loss head in the flowpath fluctuates and increases on the basis of the shape change of the flowpath section, and the loss head or pressure loss increases along the flowpath length. The pressure loss in the flowpath also increases due to the increase of the loss head, and it is difficult to smoothly move and pass the fluid. As a result, a pressure means such as a pump to apply pressure to the fluid in the flowpath might be required.

However, in a constitution comprising such a pump, the micro fluid device itself is complicated and enlarged, and it is difficult to constitute the passive type micro fluid device 1 comprising the capillary pump flowpath 6 to move the fluid by the capillary action or the like without applying any pressure from the outside as described above.

Thus, in the mixer flowpath 7 shown in FIG 9, the shape of the flowpath section is changed to vary the position of the figure center in the flowpath section, but the periodic repetition structure of the flowpath is set and constituted in a predetermined shape so that the loss head does not fluctuate or increase due to the change of the flowpath section.

In consequence, even in the constitution where the figure center of the flowpath section is changed to efficiently mix the fluids, when the loss head in the flowpath is prevented from fluctuating, the increase of the loss head or the pressure loss in the flowpath is avoided, and it is possible to smoothly pass and move the fluids in the flowpath while mixing the fluids, without requiring any pressure means such as the pump.

Specifically, in the mixer flowpath 7 shown in FIG. 9, a shape and a dimension are set to obtain the following shape.

[Generation of Loss in Flowpath (Loss Head)]

When there is a steady flow of an incompressible perfect fluid, Bernoulli's Equation 1 is established along a flowline as follows.

[Equation 1]

$$\frac{p}{r} + \frac{v^2}{2g} + z = H \text{ (constant)}$$

ρ: pressure
v: flow velocity
y: specific weight of the fluid
g: acceleration of gravity
z: height from a reference horizontal surface

Equation 1 mentioned above is established along the one flowline (the flowpath), and hence a value of the above H varies with each flowpath.

Furthermore, generation of friction or another loss in the flowpath can be represented in Equation 2 mentioned below by use of Equation 1 mentioned above. In Equation 2, an upstream section (section 1) and a downstream section (section 2) are taken from the flowpath, and values on both the sections are represented by subscripts 1 and 2. Furthermore, in Equation 2 below, h indicates loss generated between two sections, and is generally called the loss head (between the two sections).

[Equation 2]

$$\underbrace{\frac{p_1}{r} + \frac{v_1^2}{2g} + z_1}_{H_1} = \underbrace{\frac{p_2}{r} + \frac{v_2^2}{2g} + z_2}_{H_2} + h$$

Furthermore, Equation 2 mentioned above can be arranged and generally represented by Equation 3 mentioned below.

At this time, the loss head h increases toward the downstream section of the flowpath.

[Equation 3]

$$\frac{p}{r} + \frac{v^2}{2g} + z + h = H \text{ (constant)}$$

Here, as to loss (the loss head h) generated in the flow of the fluid in the flowpath, when the fluid flows through a straight circular tube of the flowpath which has an inner diameter d (m) at an average flow velocity v (m/s), the loss head h (m) to a length l (m) of the flowpath can be represented by Equation 4 mentioned below by use of a dimensionless coefficient $\lambda$.

[Equation 4]

$$h = \lambda \frac{l}{d} \frac{v^2}{2g}$$

In Equation 4 mentioned above, the coefficient $\lambda$ and the gravity acceleration g are unchangeable in any section of the flowpath. Therefore, when the flowpath length l is the same, the inner diameter d (m) of the flowpath and the average flow velocity v (m/s) of the fluid are factors for the fluctuation (increase) of the loss head h.

Thus, in the present embodiment, Equation 4 mentioned above is arranged to establish Equations 5 and 6 mentioned below, and the sectional shape of the flowpath is set so that the flowpath satisfies Equations 5 and 6 in changing the figure center of the flowpath section along the flowpath axial direction.

[Equation 5]

$$a = \frac{Um^2}{de}$$

[Equation 6]

$$\left| \frac{a - \overline{a}}{\overline{a}} \right| < 0.1$$

a: loss head due to the shape change
Um: average flow velocity
de: equivalent diameter
$\overline{a}$: average value of a

Thus, the shape of the flowpath is set and formed to satisfy conditions of Equations 5 and 6, thereby suppressing increase/decrease of the loss head due to the change of the sectional shape of the flowpath within a predetermined range ($\pm$10%), even when the sectional shape of the flowpath is formed to change and vary the figure center along the flowpath axial direction.

In a state where the above conditions of Equations 5 and 6 are satisfied and the loss head of the flowpath scarcely fluctuates, the loss head is prevented from being generated in the same manner as in a straight flowpath (a circular tubular flowpath) having a diameter corresponding to the equivalent diameter de (= 4 A/S (A: an area of the subject figure and S: peripheral length of the subject figure).

That is to say, the flowpath which satisfies the above conditions of Equations 5 and 6 is equivalent to the straight flowpath, i.e., a flowpath in which the increase of the loss head is suppressed down to the same amount as in a case where the mixer flowpath is not disposed but the straight flowpath is disposed. Therefore, the loss head of the flowpath does not particularly increase owing to the change of the figure center of the flowpath section, as long as the conditions of Equations 5 and 6 are satisfied.

In consequence, the fluid can smoothly be moved and passed in the same manner as in the straight flowpath while mixing and kneading the fluid in accordance with the change of the figure center, without requiring any pressure means such as the pump, and this constitution is suitably compatible with and applicable to the passive type micro fluid device 1.

It is to be noted that in the present embodiment, the flowpath is set to be equivalent to the straight flowpath on the basis of the loss head in the flowpath as shown in Equations 1 to 6 mentioned above.

Alternatively, the setting does not have to be based on the loss head, the flow resistance in the flowpath may directly be calculated, and the flowpath in which there are not any fluctuations in the flow resistance can be set on the basis of the flow resistance itself.

That is to say, the flow resistance of the mixer flowpath 7 is directly calculated and set, and hence it is possible to form the flowpath which is equivalent to the straight flowpath in the same manner as in the case where the flowpath is based on the above-mentioned loss head.

Here, the flow resistance in the rectangular flowpath is obtainable as follows.

[Equation 7]

$$Q = \frac{1}{\eta} \frac{P_c}{R_F}$$

[Equation 8]

$$R_F = \left[ \frac{1}{12} \left( 1 + \frac{5}{6} \frac{a}{b} \right) \frac{abR_H^2}{L} \right]^{-1}$$ (in a case where a > b)

$$R_F = \left[ \frac{1}{12} \left( 1 + \frac{5}{6} \frac{b}{a} \right) \frac{abR_H^2}{L} \right]^{-1}$$ (in a case where a < b)

Q: flow rate
$\eta$: viscosity
Pc: capillary pressure
$R_F$: flow resistance
a: flowpath depth
b: flowpath width
$R_H$: hydraulic radius
L: flowpath length

[Flowpath Shape]

Next, description will be made as to a specific shape of the mixer flowpath 7 formed so that the loss head (or the flow resistance) falls within a predetermined range to satisfy the above conditions of Equations 5 and 6 (or Equations 7 and 8) with reference to FIG 9.

As shown in FIG. 9, the mixer flowpath 7 concerned with the present embodiment comprises a constitution where the figure center of the flowpath section continuously changes.

Initially, as shown in FIG 9(a), the mixer flowpath 7 is formed so that the flowpath width continuously varies to increase and decrease at predetermined intervals, in a plan view of an XY-plane seen from the side of the upper surface of the micro fluid device 1.

Specifically, the mixer flowpath 7 is formed so that the flowpath width in the XY-plane varies continuously and periodically in a range of 15 μm to 60 μm at the interval of, for example, a flowpath length of 2.5 mm (X = 0.0 mm to 2.5 mm).

Furthermore, as shown in FIG. 9(b), the mixer flowpath 7 is formed so that the flowpath depth continuously varies to increase and decrease at predetermined intervals, in a side view of a ZX-plane of the flowpath section along the flowpath axial direction.

Specifically, the mixer flowpath 7 is formed so that the flowpath depth in the ZX-plane continuously and periodically varies in a range of 20 $\mu$m to 120 $\mu$m along a sine curve at the interval of, for example, the flowpath length of 2.5 mm (X = 0.0 mm to 2.5 mm).

In the mixer flowpath 7 comprising this constitution, the sectional shape continuously changes in each of an XY-direction and a ZX-direction so that along the flowpath axial direction from the left side of the drawing, the section has, for example, "a width of 20 $\mu$m $\times$ a depth of 60 $\mu$m" at X = 0.0 mm, "a width of 60 $\mu$m $\times$ a depth of 20 $\mu$m" at X = 0.6 mm, "a width of 15 $\mu$m $\times$ a depth of 120 $\mu$m" at X = 1.8 mm, and "a width of 20 $\mu$m $\times$ a depth of 60 $\mu$m" at X = 2.5 mm.

Thus, these values of the width and depth of the mixer flowpath 7 are calculated and set to satisfy the above-mentioned conditions of Equations 5 and 6, i.e., so that the loss head falls within the predetermined range ($\pm$10%).

It is to be noted that as to the above-mentioned width and depth of the mixer flowpath 7 which satisfies the above conditions of Equations 5 and 6 based on the loss head, the calculation and setting can be performed by using a known simulator or the like.

Furthermore, in the mixer flowpath 7 which satisfies the above conditions of Equations 5 and 6, the size (the width and depth) of the flowpath, the flowpath length, the flowpath shape and others can arbitrarily be set in accordance with the use application of the micro fluid device 1, the kind of fluid, and the like.

As described above, in the mixer flowpath 7 concerned with the present embodiment, the flowpath width in the XY-plane and the flowpath depth in the ZX-plane continuously and periodically vary.

Consequently, the figure center of the flowpath section of the mixer flowpath continuously changes in a vertical direction (an upward-downward direction in the drawing) along the flowpath axial direction.

Furthermore, owing to this change of the figure center, the velocity of the fluid is generated and changed in the vertical and horizontal directions in the flowpath.

Consequently, in the present embodiment, the mixer flowpath 7 is formed in the periodic repetition structure, thereby changing the figure center of the flowpath section along the flowpath axial direction, and the periodic repetition structure in the flowpath can be set to avoid the fluctuation of the loss head in the flowpath owing to the change of the sectional shape of the flowpath.

Thus, such a flowpath structure is employed, thereby generating and changing the velocity of the fluid flowing in the flowpath, in the vertical and horizontal directions of the flowpath in accordance with the change of the figure center of the flowpath section. In consequence, the fluid in the flowpath is mixed and kneaded in the vertical and horizontal directions of the flowpath.

Furthermore, the shape of the flowpath is set so that the loss head in the flowpath does not fluctuate even when the figure center of the flowpath section changes in this way. Therefore, the increase of the loss head or the pressure loss in the flowpath can be suppressed down to the same amount as in the case where the mixer flowpath is not disposed but the straight flowpath is disposed, and hence the fluids can be passed and moved in the flowpath while mixing the fluids, without requiring any pressure means such as the pump.

Therefore, according to the mixer flowpath 7 concerned with the present embodiment, the fluids can reliably and efficiently be mixed with the constitution of the flowpath itself, and hence the fluids can be mixed without requiring any pressure means such as the pump and without excessively complicating nor prolonging the flowpath.

In consequence, the suitable mixer flowpath 7 is achievable as the passive type micro fluid device, for example, a simple and quick diagnosis kit for influenza.

Additionally, the mixer flowpath 7 having an effect that the fluids can efficiently be mixed and kneaded by this simple and short flowpath structure produces an effect similar to the above effect irrespective of the size of the flowpath, and hence the minute chambers to mix the fluids are not required to be formed along the complicated long pathway as disclosed in Patent Literature 2 mentioned above, so that the size (the width and the depth) of the flowpath can be set to be large.

Therefore, in the present embodiment, as to the mixer flowpath 7 including the detection flowpath 8 disposed on the downstream side of the mixer flowpath, each of the flowpath width and the flowpath depth is set to the length (the size) of 30 $\mu$m or more so that the flowpath width and the flowpath are sufficiently larger than the diameter of the labeling substance in the same manner as in the capillary pump flowpath 6 mentioned above.

In consequence, even when the labeling substance having the large diameter of, for example, 400 nm or more is used as the labeling substance, it is possible to reliably prevent the clogging with the labeling substance and the slowdown, stop or the like of the liquid sending in the mixer flowpath 7 and the detection flowpath 8, and the large-diameter labeling substance can improve its visibility and signal strength in the antigen-antibody reaction.

It is to be noted that also as to the mixer flowpath 7, similarly to the capillary pump flowpath 6 mentioned above, the flowpath width and the flowpath depth are not required to be 30 $\mu$m or more in all areas, as long as the flowpath width and the flowpath depth are larger than at least the diameter of the labeling substance. However, it is more preferable that the flowpath width and the flowpath depth are 30 $\mu$m or more in all the areas of the mixer flowpath 7.

[Mixer flowpath comprising a plurality of grooves]

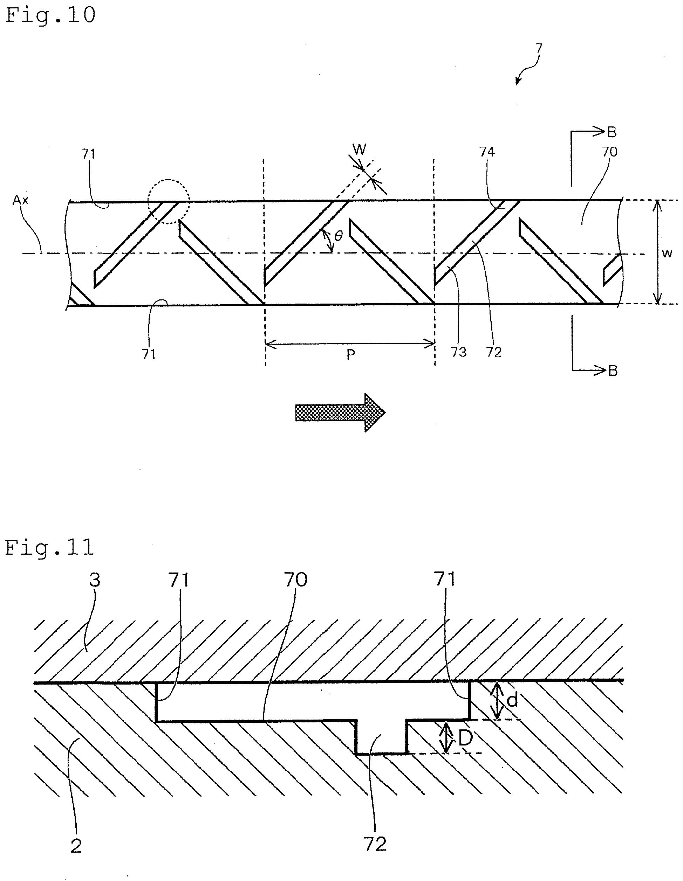

As the mixer flowpath 7 of the present embodiment, such a mixer flowpath 7 as shown in FIGS. 10 to 13 can be employed in place of or together with the mixer flowpath 7 in which the figure center of the flowpath section is changed as described above.

FIG 10 is an enlarged plan view of a substantial part showing another example of the mixer flowpath 7 of the micro fluid device 1 concerned with the present embodiment, and shows the enlarged substantial part of the mixer flowpath 7. It is to be noted that the drawing shows a flowpath axis Ax with a dot-dash line passing the center of the flowpath in a width direction, and shows a moving direction of the fluid with an arrow.

Additionally, FIG 11 is a sectional view cut along the B-B line of the mixer flowpath shown in Fig. 10.

As shown in the drawings, in the mixer flowpath 7, there is employed a constitution where a plurality of grooves 72 inclined to extend in the flowpath axial direction are arranged along the flowpath axial direction in a flowpath bottom surface 70.

Each of the grooves 72 arranged in the flowpath bottom surface 70 has an upstream end 73 and a downstream end 74, and a part of the fluid moving in the flowpath flows into the groove from the upstream end 73.

A part of the fluid flowing into the groove 72 flows around to a side lower than a fluid moving above the groove 72, and proceeds in the groove 72 to cross a flow of the upper fluid. Then, the fluid reaching the downstream end 74 to lose its passage jets out from the downstream end 74 while forming an upward flow (a plume), and joins the upper moving fluid to behave so that the fluids are mixed in each other.

To bring about this behavior in the fluids which move in the flowpath, it is preferable that an inclination angle $\theta$ of the groove 72 to the flowpath axial direction is from 10 to 80°. When the inclination angle is less than 10°, the number of the grooves per unit length of the flowpath decreases and hence a mixing efficiency decreases, and when the inclination angle $\theta$ is larger than 80°, the pressure loss might increase.

Thus, in the mixer flowpath 7 shown in FIG. 10, the above-mentioned behavior of the fluid is repeated for each of the grooves 72 arranged along the flowpath axial direction so that an inclination direction reverses at a regular or irregular period, thereby generating micro plumes in the fluids moving in the flowpath, to mix the fluids.

As described above, to mix the fluids moving in the flowpath, the inclination directions of the grooves 72 alternately reverse in the example shown in FIG. 10. According to this configuration, the micro plumes are alternately generated in the fluids moving in the flowpath, and the fluids moving in the flowpath can more efficiently be mixed.

Furthermore, when the grooves 72 are arranged in this configuration, it is preferable to arrange the grooves 72 in consideration of arrangement spaces of the grooves 72 so that the downstream end 74 of the groove 72 on the upstream side is positioned away from the upstream end 73 of the groove 72 adjacent to the downstream side of the groove 72 along the same line perpendicular to the flowpath axial direction.

Additionally, in the example shown in FIG. 10, each of the grooves 72 is formed to extend across a central portion of the flowpath bottom surface 70 in the width direction to a flowpath side wall 71.

According to this configuration, a range where the micro plumes are generated can widen in the flowpath width direction. Consequently, the mixing of the fluid moving on one side of the flowpath width direction with the fluid moving on the other side thereof can be prompted to more efficiently mix the fluids moving in the flowpath.

In addition, when the fluid jets out from the downstream end 74 of the groove 72 while forming the upward flow, it is preferable that the downstream end 74 of the groove 72 is formed at an acute angle.

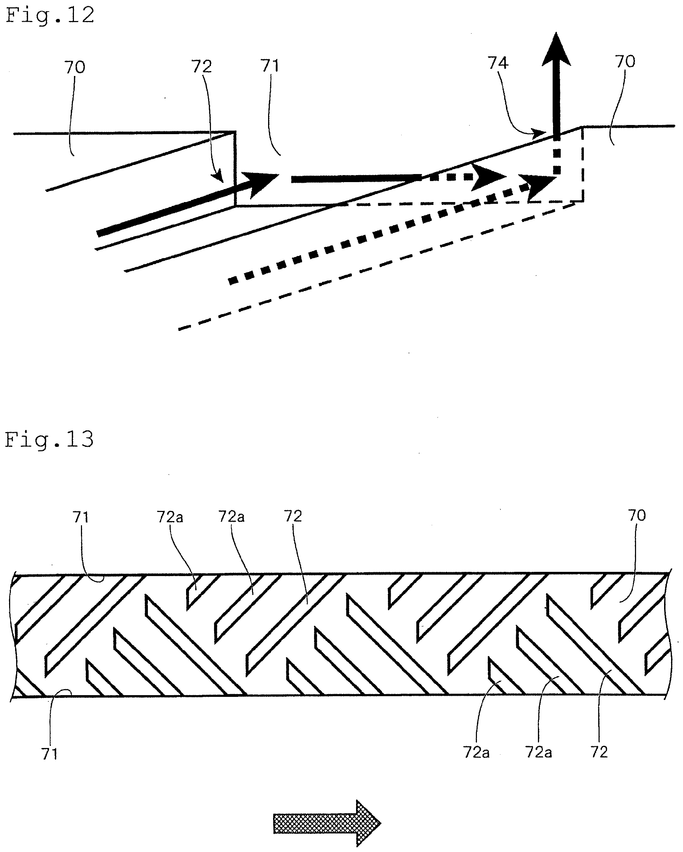

Thus, the downstream end 74 is formed at the acute angle. Consequently, as shown in FIG. 12, the fluid proceeding in the grooves 72 is concentrated on a tip of the downstream end 74, and the fluid reaching the downstream end 74 jets out from the groove while forming a larger upward flow, and is mixed with another fluid moving above the fluid over a broad range, so that the fluids moving in the flowpath can more efficiently be mixed.

Here, FIG. 12 is a schematic perspective view of the downstream end 74 of the groove 72 shown in a part surrounded with a chain line in FIG. 10, and FIG. 12 shows the flow of the fluid with arrows.

As apparent from this drawing, the fluid reaching the downstream end 74 jets outside while forming the upward flow along the flowpath side wall 71.

On the other hand, it is preferable that the upstream end 73 of the groove 72 is formed perpendicularly to the flowpath axial direction so that the fluid moving in the flowpath easily flows into the groove 72.

Furthermore, in the present embodiment, as a modified example of the mixer flowpath 7 comprising a plurality of grooves 72 mentioned above, at least one (two in the example shown in the drawing) sub groove 72a may be formed in parallel with a groove 72 adjacent to the downstream side of an upstream groove 72 and along an extending direction of the upstream groove 72 as shown in FIG. 13. An arrow in FIG. 13 shows a moving direction of the fluid in the same manner as in FIG. 10.

In this case, the sub groove 72a similar to the groove 72 can be formed to have a suitably adjusted length, in an area corresponding to a dead space where the groove 72 is not formed in the example shown in FIG. 10.

Consequently, the grooves 72 and the sub grooves 72a are densely arranged on the flowpath bottom surface 70 to improve space utilization, and more micro plumes can be generated in the fluids moving in the flowpath, to further efficiently mix the fluids.

Then, according to the mixer flowpath 7 comprising the plurality of grooves 72 as described above, the fluids can efficiently be mixed and kneaded by a simple and short flowpath structure and this effect is generated irrespective of the size of the flowpath, in the same manner as in the mixer flowpath 7 in which the figure center of the flowpath section shown in FIG. 9 is changed. Consequently, the mixer flowpath as well as the detection flowpath 8 disposed on the downstream side can be formed to set each of the flowpath width and the flowpath depth to a length (a size) of, for example, 30 $\mu$m or more so that the flowpath width and the flowpath depth are sufficiently larger than the diameter of the labeling substance.

In consequence, the visibility and signal strength of the specimen in the antigen-antibody reaction can be enhanced by using the labeling substance having a large diameter of, for example, 400 nm or more, and a reliable influenza diagnosis can be performed.

It is to be noted that also as to the mixer flowpath 7 comprising the plurality of grooves 72, similarly to the mixer flowpath 7 shown in FIG. 9, the flowpath width and the flowpath depth are not required to be 30 $\mu$m or more in all areas, as long as the flowpath width and the flowpath depth are larger than at least the diameter of the labeling substance. However, it is more preferable that the flowpath width and the flowpath depth are 30 $\mu$m or more in all the areas.

[Labeling Substance]

As described above, in the micro fluid device 1 of the present embodiment, the flowpath width and flowpath depth of each of the flowpaths 6, 7 and 8 to move and send the specimen and the labeling reagent can be set to be much larger than those in a conventional immunochromatographic device, and as a result, the respective flowpaths can be formed so that the size of the flowpath section is sufficiently larger than the diameter of the labeling substance.

Furthermore, in the present embodiment, such flowpath characteristics are utilized, and the labeling substance having a predetermined diameter or more is usable as the labeling substance in which the labeling antibody combining with the influenza antigen contained in the specimen dropped onto the conjugate pad 4 is solidified.

Consequently, in the present embodiment, a size of the labeling substance is set to a necessary and sufficient size of the labeling substance in consideration of the visibility and signal strength in the antigen-antibody reaction. cerned w

Specifically, beads having a diameter of 400 nm or more are usable as the labeling substance.

By use of the large-diameter beads, it is possible to sufficiently enhance the visibility and the signal strength in the detection flowpath of the micro fluid device 1, and when the size (the flowpath width/depth) of each of the flowpaths 6, 7 and 8 formed on the substrate 2 is set to about 10 $\mu$m, it is possible to reliably prevent, for example, occurrence of the clogging with the labeling substance or piling-up of the labeling substance in the flowpath.

In the conventional immunochromatographic device where a foam such as the nitrocellulose foam or a fibrous material is used as the flowpath of the specimen, to avoid a problem that the labeling substance clogs porous areas of the foam or the like which is the flowpath, it has been necessary that the particle diameter of the labeling substance is sufficiently small. For example, an average pore diameter of the nitrocellulose foam is about 10 $\mu$m, and in consideration of variable pore diameters of the foam, the size of the labeling substance is required to be 400 nm or less at the maximum. Therefore, for example, gold colloid having a particle diameter of about 40 nm is usually used as the labeling substance. Consequently, by use of the labeling substance having the particle diameter of about 40 nm, a specimen capture section of the device does not sufficiently develop a color because the particle diameter is small, and the visibility and signal strength deteriorate. Particularly, when the concentration of the analyte contained in the specimen is low, the device does not develop the color, and overlooking or wrong judgment might be caused.

To eliminate the problem, in the present embodiment, the flowpath structure having the above-mentioned strong driving force and fluid mixing performance is employed, and the large-diameter beads which have the diameter of 400 nm or more and which have been unusable in the conventional immunochromatographic device are used as the labeling substance.

In consequence, the labeling substance comprising large particles having a diameter which is 10 times as much as and an area which is 100 times as much as those of a labeling substance such as the gold colloid for the immunochromatographic device is usable, suitable visibility and signal strength are obtainable, and the judgment accuracy of the influenza infection can be enhanced.

Here, as the large-diameter beads constituting the labeling substance concerned with the present embodiment, there is usable, for example, polystyrene, silica, acryl, quartz, chitosan, dextran, albumin, agarose, polylactic acid (PLA), polyethyleneimine, aluminum oxide, borosilicate glass, soda-lime glass, PLGA, iron oxide, palladium, or the like.

Furthermore, the above-mentioned large-diameter beads can obtain the suitable visibility and signal strength, and additionally have an effect of contributing to enhancement of the fluid mixing performance.

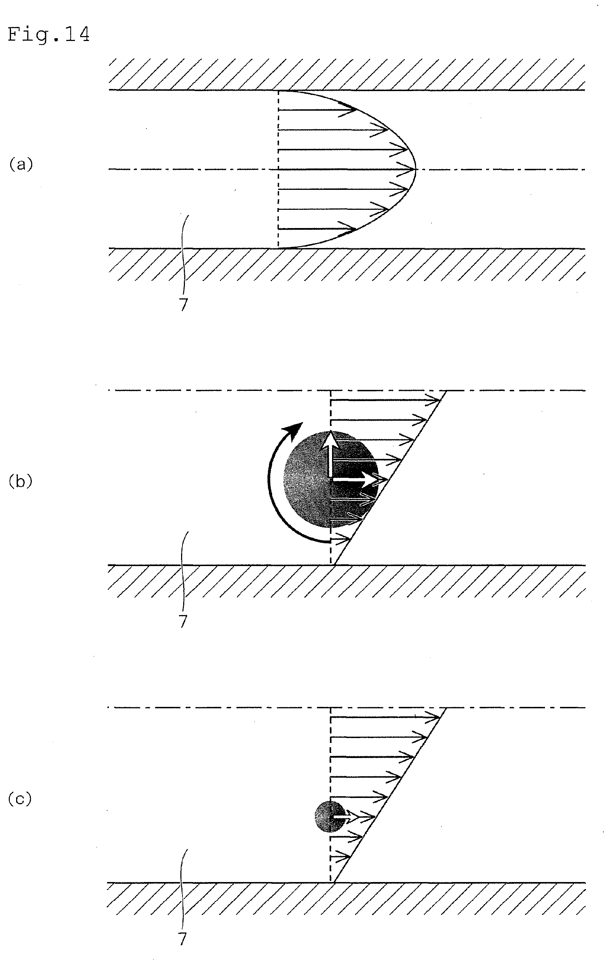

As shown in FIG. 14(a), when there is the steady flow of the incompressible perfect fluid in each flowpath of the micro fluid device 1, velocity components are generated in the form of a parabola which swells out in a proceeding direction, along the flowpath axial direction in the fluid.

Thus, in the steady flow, the velocity at the center of the flowpath is highest, and as the flow is distant from the flowpath center, the velocity decreases. Therefore, in a case of a grain having a regular size or more to the flowpath width, e.g., the large-diameter bead having the particle diameter of 400 nm or more, the velocity of the fluid varies with regions of the grain as shown in FIG. 14(b). As a result, the grain rotates, thereby generating a lift force in a direction which crosses the flow direction of the fluid.

Thus, the rotation and the lift force are generated. Consequently, the grain in the fluid repeats moving also in the direction (an upward-downward direction in the drawing) which crosses the flow direction of the fluid, while proceeding in the flow direction of the fluid. As a result, mixing of the fluids occurs and is promoted.

On the other hand, in a case of a micro grain having a particle diameter of about 40 nm as in the gold colloid used in the usual immunochromatographic device, as shown in FIG. 14(c), the above-mentioned difference in the velocity component of the fluid does not affect the grain and any rotary force or any lift force is not generated in the grain, because the grain is much smaller than the flowpath width. Therefore, the grain never contributes to the mixing of the fluids.

Consequently, the employment of the large-diameter beads concerned with the present embodiment can not only enhance the visibility and signal strength in the antigen-antibody reaction but also promote the mixing of the fluids, and hence, the beads are more suitable as the labeling substance for use in the immunological measurement device.

Therefore, by use of the large-diameter beads, it is possible to further enhance the above-mentioned mixing performance of the mixer flowpath 7 concerned with the present embodiment, and it is possible to mix the fluids by the labeling substance itself also when there is used the usual straight flowpath which does not involve the figure center change or does not comprise the plurality of grooves.

[Bonding Method]

Next, description will be made as to a method of bonding base materials to each other in a case where the substrate 2 and the cover body 3 are the base materials made of the resin in the micro fluid device 1 mentioned above, with reference to FIG. 15.

The substrate 2 and the cover body 3 which are made of the resin can be bonded by using the following bonding method.

That is to say, in the present embodiment, when the two resin base materials constituting the substrate 2 and the cover body 3 are bonded to manufacture the micro fluid device 1, the base materials can be bonded by a method comprising a step of applying high energy to a bonded surface of at least one of the two resin base materials, to flatten and soften the bonded surface, and a step of laminating the two resin base materials and then heating and/or pressurizing and bonding the two resin base materials.

Specifically, in the bonding method concerned with the present embodiment, initially as shown in FIG. 15(a), the high energy is applied to base material surfaces constituting the respective bonded surfaces of the substrate 2 in which the flowpaths 6, 7 and 8 of the micro fluid device 1 are formed, and the cover body 3 which is the lid member to be laminated on the substrate 2.

When the high energy having a large atomic weight is applied to the surfaces of the resin base materials, the base material surfaces can be modified, and specifically, it is possible to flatten and soften the base material surfaces.

Due to the flattening and softening, contact properties and adhering properties of the base material surfaces to each other can be enhanced, and even at a lower bonding temperature, it is possible to firmly fuse and bond the materials by a van der Waals force.

Here, in the present embodiment, as shown in FIG. 15(a), an argon plasma is applied as the high energy to the bonded surfaces of the resin base materials.

The argon plasma has a large atomic weight, and an argon gas which is easily turned to the plasma is introduced as a raw material gas to discharge electricity, thereby generating Ar ions or Ar radicals which are active species of argon by means of the plasma. Furthermore, when the argon active species having the large atomic weight and a strong attack power collide with the surfaces of the resin base materials, molecules of the resin base materials can be cut apart from each other.

In consequence, the surfaces of the resin base materials can be modified, and specifically, the base material surfaces are flattened, and molecular weights of the base material surfaces are lowered, i.e., the surfaces are softened.

Thus, the flattened and softened (by lowering the molecular weights) surfaces are considered as the bonded surfaces. Consequently, the contact properties and adhering properties of the base material surfaces to each other can be enhanced, and both the surfaces can firmly be fused and bonded also at a lower bonding temperature.

In consequence, as shown in FIG. 15(b), it is possible to bond the resin base materials (the substrate 2 and the cover body 3) also at a temperature which is not higher than a glass transition point or a melting point. For example, it is possible to bond two base materials at a low bonding temperature of about 30°C.

Then, the base materials are bonded at the temperature which is not higher than the glass transition point or the melting point. Consequently, the method is suitably usable as a manufacturing method of the micro fluid device 1 having a high reliability, without causing, for example, deformation of each of the flowpaths 6, 7 and 8 formed on the substrate 2.

It is to be noted that as shown in FIG. 15(b), the bonding method of the present embodiment enables the bonding at a bonding temperature of about 30°C. Therefore, at least one of the heating and the pressurizing may be performed. For example, the resin base materials can be bonded only by performing the pressurizing without performing the heating. Alternatively, the resin base materials can be bonded only by performing the heating without performing the pressurizing.

However, for the purpose of more firmly and reliably bonding the resin base materials to each other, it is preferable that the heating and the pressurizing are performed at an appropriate temperature and an appropriate pressure.

Here, in the present embodiment, as the high energy to be applied to the bonded surfaces of the resin base materials, the above-mentioned argon plasma is preferable, but the present invention is not limited to this example.

For example, as the application of the high energy other than the argon plasma, either one of an oxygen plasma, a mixed plasma of argon with oxygen or the like and a vacuum ultraviolet ray may be applied.

This application of the energy which is easily turned to the plasma and has the strong attack power is preferable in modifying the resin base material surfaces, i.e., flattening and softening (lowering the molecular weight of) the base material surfaces in the same manner as in the above-mentioned argon plasma application, and this energy can be employed in place of the argon plasma.

Furthermore, this high energy may be applied to at least one of the bonded surfaces of the two resin base material to be bonded. However, for the purpose of obtaining a stronger bonding strength, it is preferable to apply the high energy to each of the bonded surfaces of the two resin base materials to be bonded.

According to the bonding method of the present embodiment, the bonded surfaces of the resin base materials to be bonded are modified to be flattened and softened. Consequently, the bonded surfaces made of the resin can be thermally fused to each other at a low bonding temperature which is not higher than the glass transition point or the melting point.

Owing to the low-temperature bonding, the micro fluid device 1 comprising a desirable flowpath space can precisely be manufactured, without causing, for example, deformation of the micro flow paths minutely formed on the substrate 2 made of the resin by the heating at the high temperature.

As described above, according to the immunological measurement device comprising the micro fluid device 1 concerned with the present embodiment, a strong capillary action is applied to the flowpath itself so as to reliably move and mix the specimen and the labeling reagent, without constituting the flowpath to move and send the specimen by use of a porous material as in the conventional immunochromatographic device, and without excessively prolonging nor complicating the flowpath.

Consequently, in the respective flowpaths 6, 7 and 8 of the micro fluid device 1, the flowpath width and depth can be set to the sufficient size, and as a result, clogging, piling-up or the like does not occur in the flowpath if the particle diameter of the labeling substance is increased.

Therefore, the visibility and signal strength of the sufficiently large labeling substance can reliably and effectively be enhanced.

In consequence, the judgment in the detection flowpath 8 of the micro fluid device 1 can reliably be made, and even when the concentration of the analyte contained in the specimen of viruses or the like is low, the wrong judgment in the conventional immunochromatographic device does not occur, which enables, for example, early detection of influenza infection.

Furthermore, the reliable judgment and detection can be performed by using the low-concentration analyte, and hence, also when a specimen sampling method which impinges invasiveness and a strong pain on the person to be tested is not taken, for example, it is possible to use, as the specimen, the nasal swab which only contains the low-concentration analyte, and the immunological measurement device which is minimally invasive and easy to use is achievable.

Additionally, the micro fluid device 1 concerned with the present embodiment comprises the capillary pump flowpath 6 and the mixer flowpath 7 (the detection flowpath 8) having characteristic structures, and therefore constitutes a self-liquid sending type device which can move the fluid by the capillary action in the flowpath, without requiring any pump power from the outside.

In consequence, the micro fluid device 1 of the present embodiment does not require an external device such as the pump, and the constitution of the device itself can be miniaturized and simplified as much as possible.

Therefore, according to the micro fluid device 1 of the present embodiment, there can be provided the immunological measurement device suitable for, for example, the diagnosis kit for influenza which comprises a simple constitution but has a high reliability and is friendly also to the person to be tested.

The description has been made as to the preferable embodiment of the present invention, but needless to say, the present invention is not limited to the above-mentioned embodiment, and can variously be changed in the scope of the present invention.

For example, in the embodiment concerned with the above-mentioned micro liquid sending structure of the capillary pump flowpath 6, for the purpose of relatively decreasing the flow resistance of the low flow resistance liquid sending passage 61a so that the flow resistance is lower than that of the other liquid sending passage 61b, the edge portion 62 which develops the pinning effect is formed on the outlet side of the liquid sending passage 61 interposed between the microprojections 60 adjacent to each other via the liquid sending passage 61, and the pinning angle of the edge portion 62 is suitably set. Consequently, the flow resistance of the low flow resistance liquid sending passage 61a is relatively decreased to be lower than the flow resistance of the other liquid sending passage 61b, but the present invention is not limited to this example.

For example, the flow resistance of the low flow resistance liquid sending passage 61a may relatively be decreased to be lower than the flow resistance of the other liquid sending passage 61b as follows.

1) Only in the microprojections 60 adjacent to each other via the other liquid sending passage 61b, the edge portion 62 which develops the pinning effect is formed on an outlet side of the liquid sending passage 61b. For example, the outlet side of the low flow resistance liquid sending passage 61a interposed between the microprojections adjacent to each other via the liquid sending passage 61a is formed in a rounded shape which does not develop the pinning effect, to relatively decrease the flow resistance of the low flow resistance liquid sending passage 61a so that the flow resistance is lower than the flow resistance of the other liquid sending passage 61b.

2) A region of the microprojection 60 on the side of the other liquid sending passage 61b which is interposed between the microprojections adjacent to each other via the liquid sending passage 61b is subjected to a hydrophobic treatment to increase the flow resistance. Consequently, the flow resistance of the low flow resistance liquid sending passage 61a is relatively decreased so that the flow resistance is lower than the flow resistance of the other liquid sending passage 61b.

3) A sectional area of the low flow resistance liquid sending passage 61a is increased, to relatively decrease the flow resistance of the low flow resistance liquid sending passage 61a so that the flow resistance is lower than the flow resistance of the other liquid sending passage 61b.

Furthermore, the shape of the microprojection 60 of the capillary pump flowpath 6 is not limited to the above-mentioned embodiment, and the microprojections can suitably be designed without disturbing the effect of the present invention.

Additionally, in the above-mentioned embodiment, the description has been made as to the example where the test flowpath 8a and the control flowpath 8b which are disposed in the detection flowpath 8 of the micro fluid device 1 are formed to have a large width and a small depth and their bottom surfaces are coated with the capture antibody, but the present invention is not limited to this example. For example, the micro liquid sending structure disposed in the capillary pump flowpath 6 may be formed also in each of the test flowpath 8a and the control flowpath 8b which are disposed in the detection flowpath 8, to control the liquid sending direction of the specimen preparation passing through the flowpaths 8a and 8b. At this time, the microprojections 60 are previously coated with the capture antibody CA. Therefore, for example, the influenza antigen LA combined with the labeling reagent is more easily captured in the test flowpath 8a, and the influenza antigen LA combined with the labeling reagent is also captured along a height direction of the microprojection 60. Consequently, the color developed by the labeling substance can more easily be visually recognized.

Furthermore, in the above-mentioned embodiment, the mixer flowpath 7 comprising the plurality of grooves 72 is formed so that the linear groove 72 extends to cross the flowpath axial direction, but the configuration of the groove 72 is not limited to this example.

For example, as long as the fluid flowing into the groove from the upstream end 73 proceeds in the groove 72 to jet out from the downstream end 74 while forming the upward flow, for example, a constitution where the groove 72 is curved in an S-shape may be employed.

Additionally, in the above-mentioned embodiment, the diagnosis kit for influenza has been described as the example of the micro fluid device concerned with the present invention, but a subject to which the micro fluid device concerned with the present invention is to be applied is not particularly limited to the diagnosis kit for influenza.

Furthermore, in the above embodiment, the passive type micro fluid device which does not comprise a pressure means such as the pump to move the fluid in the flowpath has been described as the example of the constitution of the micro fluid device, but needless to say, the present invention is applicable to an active type micro fluid device comprising a pressure means such as the pump.

That is to say, in the present invention, there are not any special restrictions on the micro fluid device, as long as a plurality of fluids are required to be moved while efficiently, rapidly and reliably mixing and kneading the fluids in a minute space of a micro flowpath, and the present invention is broadly applicable regardless of a constitution, a config-

**EP 3 367 097 A1**

uration and a use purpose of the device, a kind and an amount of the fluid, and the like.

There are invoked herein all contents of the literatures described in this description and the Japanese application description on the basis of which the priority of the present application under the Paris convention is claimed.

REFERENCE SIGNS LIST

1  micro fluid device
2  substrate
3  cover body (a lid member)
4  conjugate pad
5  absorption pad
6  capillary pump flowpath
6a  first capillary pump section
6b  second capillary pump section
7  mixer flowpath (a liquid sending flowpath section)
8  detection section
8a  test flowpath
8b  control flowpath 8b

**Claims**

1. An immunological measurement device comprising a micro fluid device including a flowpath to move a specimen which is subjected to an immunological measurement by an antigen-antibody reaction, the immunological measurement device comprises:

   a labeling reagent supply unit which is disposed in an upstream section of the flowpath and labels the specimen, and a detection flowpath which is disposed downstream than the labeling reagent supply unit and measures the antigen-antibody reaction of the specimen,
   wherein the labeling reagent supply unit comprises a labeling reagent which labels the specimen by the antigen-antibody reaction and contains the labeling substance wherein an antigen or an antibody is solidified.

2. The immunological measurement device according to claim 1, wherein the flowpath comprises a self liquid sending type flowpath which moves the specimen and the labeling reagent by a capillary action in the flowpath, without requiring any pump power from the outside.

3. The immunological measurement device according to claim 1 or 2, wherein the labeling substance comprises colored beads having a large diameter of 100 nm or more.

4. The immunological measurement device according to any one of claims 1 to 3, wherein the labeling reagent supply unit comprises a conjugate pad impregnated with a labeling reagent, and the specimen is dropped onto a conjugate pad via or not via a sample pad.

5. The immunological measurement device according to any one of claims 1 to 4, which comprises a capillary pump flowpath which is disposed on at least one of upstream and downstream sides of the detection flowpath and generates a capillary action to move the specimen and the labeling reagent.

6. The immunological measurement device according to any one of claims 1 to 5, which comprises a mixer flowpath to mix the specimen and the labeling reagent, on an upstream side of the detection flowpath.

7. The immunological measurement device according to any one of claims 1 to 6, wherein a flowpath width and a flowpath depth of the flowpath are larger than a diameter of the labeling substance.

Fig.1

EP 3 367 097 A1

Fig.2

Fig.3

Fig.4

(a)

CA

8a

A                                                A

(b)

CA        3

LA        2

Fig.5

62(62b) 62(62b) 62(62a) 62(62a)

61(61b)
60(60c)
61(61b)
60(60b)
61(61a)
60(60a)
61(61a)
60(60b)
61(61b)
60(60c)
61(61b)

60(60c)
62(62b)
61(61b)
62(62b)
60(60b)
62(62a)
61(61a)
62(62a)
60(60b)
62(62b)
61(61b)
62(62b)
60(60c)

61(61a) 61(61a) 61(61a)

EP 3 367 097 A1

Fig.6

θ

α

(a)

θ+(π−α)

θ

α

(b)

26

Fig.7

Fig.8

(a)

(b)

Fig.9

(a)

Liquid sending direction

Y
X

(b)

Z
X

(c)

Z
Y
X

6b

8
8b 8a

1

6a

7

Fig.10

Fig.11

Fig.12

Fig.13

Fig.14

(a)

7

(b)

7

(c)

7

Fig.15

(a)

Argon Plasma

Plasma

Ar radicals

Ar ions

Base Materials

2       7    3

Bonding Temperature<Tg
Heating &Pressurizing

(b)

3
2      1

7

## INTERNATIONAL SEARCH REPORT

| International application No. |
| --- |
| PCT/JP2016/080917 |

| A. CLASSIFICATION OF SUBJECT MATTER |
| --- |
| *G01N33/543*(2006.01)i, *G01N37/00*(2006.01)i |

According to International Patent Classification (IPC) or to both national classification and IPC

| B. FIELDS SEARCHED |
| --- |

Minimum documentation searched (classification system followed by classification symbols)
G01N33/543, G01N37/00

Documentation searched other than minimum documentation to the extent that such documents are included in the fields searched

| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X/Y | WO 2011/108333 A1 (Konica Minolta Opto, Inc.), 09 September 2011 (09.09.2011), paragraphs [0023] to [0027]; fig. 1 (Family: none) | 1-4,6-7/5 |
| Y | JP 2014-525569 A (The Royal Institution for the Advancement of Learning/McGill University), 29 September 2014 (29.09.2014), paragraphs [0063] to [0069], [0099] to [0100]; fig. 3, 11B & WO 2013/029159 A1 paragraphs [0063] to [0069], [0099] to [0100]; fig. 3, 11B & US 2014/0332098 A1  & EP 2751021 A1 | 5 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 13 January 2017 (13.01.17) | 24 January 2017 (24.01.17) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/080917

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-069397 A (NEC Corp.), 04 March 2004 (04.03.2004), & WO 2004/036194 A1 & US 2005/0239210 A1 & CN 1675535 A | 1-7 |
| A | JP 2012-170854 A (National University Corporation Toyohashi University of Technology), 10 September 2012 (10.09.2012), (Family: none) | 1-7 |
| A | JP 2005-199245 A (Dainippon Printing Co., Ltd.), 28 July 2005 (28.07.2005), (Family: none) | 1-7 |
| P,A | JP 2016-097353 A (Toyo Seikan Group Holdings, Ltd.), 30 May 2016 (30.05.2016), (Family: none) | 1-7 |

Form PCT/ISA/210 (continuation of second sheet) (January 2015)

**REFERENCES CITED IN THE DESCRIPTION**

**Patent documents cited in the description**

- WO 3886000 A

- WO 2009501908 A